Europäisches Patentamt

European Patent Office

Office européen des brevets

(11) Veröffentlichungsnummer : **0 082 265**
**B1**

(12) **EUROPÄISCHE PATENTSCHRIFT**

(45) Veröffentlichungstag der Patentschrift :
26.06.85

(51) Int. Cl.⁴ : **A 61 C 13/225**

(21) Anmeldenummer : 82109578.3

(22) Anmeldetag : 16.10.82

(54) Befestigungssystem für eine schleimhautgetragene Prothese.

(30) Priorität : 14.11.81 DE 3145260
07.10.82 DE 3237174

(43) Veröffentlichungstag der Anmeldung :
29.06.83 Patentblatt 83/26

(45) Bekanntmachung des Hinweises auf die Patenterteilung : 26.06.85 Patentblatt 85/26

(84) Benannte Vertragsstaaten :
AT BE CH FR GB IT LI LU NL SE

(56) Entgegenhaltungen :
CH-A-   597 843
DE-B- 2 549 114
FR-A- 1 211 044
GB-A- 2 063 680
US-A- 2 112 007
US-A- 4 204 321
US-A- 4 324 549
DIE QUINTESSENZ, Nr. 10, Oktober 1974, Berlin,
Referat Nr. 5108 I.I. ZAMIKOFF " Abgestützte
Vollprothesen " Seiten 81-84

(73) Patentinhaber : Feldmühle Aktiengesellschaft
Fritz-Vomfelde-Platz 4
D-4000 Düsseldorf 11 (DE)

(72) Erfinder : Brinkmann, Egon, Dr.
Theaterwall 14
D-2900 Oldenburg (DE)
Erfinder : Putenat, Manfred
Sodensteg 57
D-2900 Oldenburg (DE)
Erfinder : Dörre, Erhard, Dr.
Talweg 14
D-7310 Plochingen (DE)

(74) Vertreter : Uhlmann, Hans, Dr. rer.nat., Dipl.-Chem.
Gladbacher Strasse 189
D-4060 Viersen 1 (DE)

## Beschreibung

Die vorliegende Erfindung betrifft ein Befestigungssystem für eine herausnehmbare schleimhautgetragene Prothese, bestehend aus zwei auf Implantaten oder natürlichen Zahnwurzeln befestigten, das Volumen einer Halbkugel übertreffenden und mit einer hochglanzpolierten Oberfläche versehenen Kugelköpfen zum Festhalten der Prothese, aus zwei zum Eingriff mit den Kugelköpfen vorgesehenen, diesen angepaßten, und in Aushöhlungen der Prothese befestigten Aufnahmekappen aus glattem und elastischem Kunststoffmaterial und aus zwei im Bereich der Öffnung der Aufnahmekappen angeordneten Adaptionsringen.

Es sind Gebißprothesen bekannt, die ihren Halt durch Saugwirkung am Gaumen erhalten oder durch Halteelemente am Restzahnbestand gehalten werden. Diese Prothesen können als schleimhautgetragene Prothesen bezeichnet werden. Oftmals sitzen diese nicht zufriedenstellend und lösen sich während der Nahrungsaufnahme und beim Sprechen. Es wurde deshalb auch bereits vorgeschlagen, künstlichen Zahnersatz auf im Kieferkamm eingesetzten, enossalen Implantaten zu befestigen. Diese Technik hat in der Zahnheilkunde bereits eine weite Verbreitung gefunden, kann aber nicht in jedem Fall mit Erfolg angewendet werden, sondern ist im wesentlichen darauf beschränkt, nicht herausnehmbaren Einzelzahnersatz oder verkürzten Zahnreihen festsitzenden Halt zu verleihen, und wird für diesen Zweck mit zum Teil beachtlichen Erfolgen eingesetzt.

Zur Befestigung einer schleimhautgetragenen Prothese ist durch die CH-A-597 843 ein Befestigungssystem bekanntgeworden, bei dem eine Prothese, in der eine Aufnahmekappe aus elastischem Material angebracht ist, auf Kugelköpfen tragenden Implantaten befestigt wird. Die Aufnahmekappen umfassen die Kugelköpfe nahezu vollständig, wodurch eine starre Befestigung vom Druckknopftyp entsteht. Der Nachteil dieses Vorschlags liegt darin, daß eine seitliche Bewegung der Prothese durch den starren Sitz nicht möglich ist.

Aus der US-A-4 204 321 ist ein Befestigungssystem der eingangs genannten Art bekannt, bei dem die Prothese durch eine Art Druckknopftechnik einen festen Sitz erhalten soll. Nachteilig ist dabei die unzureichende Beweglichkeit der Gebißprothese, so daß dieser Vorschlag vorwiegend für eine Befestigung einer Oberkieferprothese dienen kann.

Gemäß einem in der FR-A-12 11 044 enthaltenen Vorschlag ist die Befestigung von Kugeln oder Zylindern aus rostfreien nichtmagnetischen Metallen oder Legierungen auf schraubförmigen Implantaten vorgesehen. Die vorgeschlagene Zylinderform sieht am Ende, mit dem der Zylinder auf dem Implantat befestigt wird, die Form einer Kugelkalotte vor. Zur Befestigung auf den Kugeln oder Zylindern sind entsprechend ausgebildete Gegenstücke — sogenannte Patrizen — vorgesehen, die durch Metallbänder miteinander verschweißt sind und dadurch zu einer starren Verbindung führen. Damit entsteht eine herausnehmbare Brücke. Zur Befestigung des Zahnersatzes dienen besondere stangenartig ausgebildete Stücke, die auf den über die Kugeln bzw. Zylinder geschobenen Gegenstücken befestigt sind oder die Abschlußplatte eines solchen Gegenstückes. Nachteilig bei diesem System ist es, daß infolge der starren Verbindung die beim Kauen auftretenden Kräfte direkt auf den Kugelkopf bzw. auf das Implantat weitergeleitet werden. Ein weiterer Vorschlag der FR-A-12 11 044 sieht daher eine in den Gegenstücken angeordnete elastische Masse vor. Der entscheidende Nachteil dieses Vorschlags liegt aber ebenfalls darin, daß eine seitliche Bewegung des Zahnersatzes nicht möglich ist, vielmehr eine starre Verbindung resultiert.

Auch ein in der US-A-1 101 810 beschriebenes Befestigungssystem hat den Nachteil, daß eine seitliche Bewegung der schleimhautgetragenen Prothese nicht möglich ist. Bei diesem älteren Vorschlag ist in die Prothese eine kugelförmig ausgebildete Federklemme eingelassen, die eine, mit einem Stift in einer Zahnwurzel befestigte Vollkugel übergreift. Ein weiterer Nachteil der Vollkugel liegt darin, daß die Kugel weit in die Mundhöhle hineinragt und die Prothese daher eine größere Kippneigung hat. Ein weiterer Nachteil, wie auch anderer aus dem Stand der Technik bekanntgewordener Vorschläge, besteht darin, daß mit einer Vollkugel ein Abschluß einer im Kiefer angelegten Öffnung nicht möglich ist und, z. B. Endplatten zum Schließen einer Zahnwurzelöffnung Verwendung finden müssen.

Man hat auch bereits versucht, mit Hilfe von in den Kieferkamm eingesetzten Implantaten bzw. Stegen, die an aus dem Kieferkamm herausragenden runden oder eckigen Implantatpfosten befestigt sind, einen verbesserten Halt von herausnehmbarem Zahnersatz wie z. B. Unterkiefer-Gebißprothesen zu erzielen.

Insbesondere bei der Versorgung des zahnlosen Unterkiefers sind diese Versuche jedoch wenig erfolgreich verlaufen. Die dabei auftretenden Schwierigkeiten sind wie folgt begründet: Zumeist bestehen infolge fortgeschrittener Atrophie im lateralen Anteil des Kieferknochens äußerst ungünstige anatomische Verhältnisse, so daß das Einsetzen von Implantaten in diesem Bereich selten in Frage kommt. Infolgedessen steht für Implantationen hauptsächlich die regio interforaminalis des Unterkiefers zur Verfügung. Werden in diesem Bereich eingesetzte und mit Stegen verbundene Implantate zum Halten einer Unterkiefer-Gebißprothese verwendet, resultiert eine mehr oder weniger starre Verbindung.

Infolge der Resilienz der lateralen Schleimhaut kommt es dadurch zu einer ständigen Kippbelastung während des Kauens und Sprechens. Je höher die Implantatpfosten aus dem Kieferknochen herausragen, desto mehr nimmt die Kippbelastung zu. Die Gebißprothese sitzt infolgedessen nicht ausreichend fest und verleiht dem Gebißträger ein ständiges Gefühl der Unsicherheit, da der Unterkiefer bekanntlich in der Phase der letzten Schlußbewegung eine vertikale und horizontale Bewegung ausführt, die in einer Rotationsbewegung endet, der die bisherigen Implantatsysteme bzw. der darauf befestigte, herausnehmbare Zahnersatz nicht folgen konnten, ohne daß es zu einer Fehlbelastung kam, insbesondere bei divergierend eingesetzten Implantatpfosten.

Aufgabe der vorliegenden Erfindung ist es, die bestehenden Nachteile zu beseitigen und ein Befestigungssystem für eine herausnehmbare, schleimhautgetragene Prothese zu entwickeln, durch das die Prothese, insbesondere für den totalen Zahnersatz des Unterkiefers einen festen und gegenüber seitlich wirkenden Kräften sicheren Sitz erhält. Zur Vermeidung der bekannten Nachteile soll die Prothese jedoch nicht durch eine starre Verbindung mit den Implantaten verbunden sein, vielmehr soll die Prothese beim Kauvorgang in gewisser Weise beweglich sein.

Insbesondere will die Erfindung sowohl bei geringer als auch bei extremer Lateral- und Vertikalbewegung des Kiefers einen optimalen Halt einer schleimhautgetragenen Prothese erreichen.

Eine weitere Aufgabe der vorliegenden Erfindung liegt in der Vermeidung der bekannten Nachteile, die bei Verwendung von Metallteilen in der Mundhöhle resultieren.

Schließlich sieht die Erfindung ihre Aufgabe auch darin, ein System zu schaffen, das auch dann noch einen sicheren Halt einer Prothese bewirkt, wenn diese auf divergierend stehenden Implantatpfosten befestigt werden muß.

Zur Lösung dieser Aufgabe sieht die Erfindung ein Befestigungssystem für eine herausnehmbare, schleimhautgetragene Prothese vor, das aus zwei auf Implantaten oder natürlichen Zahnwurzeln befestigten, das Volumen einer Halbkugel übertreffenden und mit einer hochglanzpolierten Oberfläche versehenen Kugelköpfen zum Festhalten der Prothese, aus zwei zum Eingriff mit den Kugelköpfen vorgesehenen, diesen angepaßten und in Aushöhlungen der Prothese befestigten Aufnahmekappen aus glattem und elastischem Kunststoffmaterial und aus zwei im Bereich der Öffnung der Aufnahmekappen angeordneten Adaptionsringen besteht und das dadurch gekennzeichnet ist, daß die Aufnahmekappen den Kugelköpfen nur im Bereich von deren Äquatoren anliegen und daß die Adaptionsringe zwischen den Wänden der Aushöhlungen und den Aufnahmekappen angeordnet sind und aus einem Kunststoffmaterial bestehen, das elastischer ist als das Material der Aufnahmekappen.

Weitere vorteilhafte Ausführungsformen der Erfindung sind durch die Unteransprüche gekennzeichnet.

Durch den Kunstgriff, daß zur Befestigung einer schleimhautgetragenen Prothese mit lediglich zwei Kugelköpfen die Aufnahmekappen der Prothese den Kugelköpfen nur im Bereich von deren Äquatoren anliegen, ergibt sich ein sicherer Sitz der Prothese, ohne daß zwischen Kugelköpfen und Prothese eine starre Verbindung besteht. Dies ist z. B. bei dem in der CH-A-597 843 beschriebenen System der Fall, wo durch das nahezu vollständige Umfassen des Kugelkopfes durch die in der Prothese angeordneten Aufnahmekappen selbst die geringste Beweglichkeit ausgeschlossen wird.

Der hervorragende Halt der Prothese bei Vertikal- und Lateralbewegungen des Kiefers ergibt sich durch die Ausbildung der Adaptionsringe aus einem Kunststoffmaterial von höherer Elastizität als das der Aufnahmekappen. Die im Bereich der Öffnungen der Aufnahmekappen zwischen den Wänden der Aushöhlungen für die Aufnahmekappen und den Aufnahmekappen angeordneten Adaptionsringe umschließen die Aufnahmekappen und bewirken deren enges Anliegen an den Kugelköpfen.

Wenn bei einseitigem Kaudruck eine der beiden Aufnahmekappen tiefer in den Kugelkopf gedrückt wird, führt zwangsläufig die andere Aufnahmekappe eine gegenläufige Bewegung aus. Während dieses Vorganges gleiten die Aufnahmekappen über wechselnde Durchmesser der Kugelköpfe. Infolge ihrer höheren Elastizität bewirken die Adaptionsringe einen in radialer Richtung auf die Aufnahmekappen einwirkenden Druck und dadurch ein enges Anliegen der Aufnahmekappen während ihrer Auf- und Abbewegung an den Kugelköpfen, ohne daß die Auf- und Abbewegung der Aufnahmekappen bzw. der Prothese in irgendeiner Weise beeinträchtigt wird. In ähnlicher Weise bewirken die Adaptionsringe auch ein enges Anliegen der Aufnahmekappen bei seitlich einwirkender Belastung.

Die vorliegende Erfindung hat damit ein Befestigungssystem geschaffen, bei dem eine mit Hilfe von Implantaten gehaltene Gebißprothese der in einer Rotationsbewegung endenden Kaubewegung eines Unterkiefers folgen kann, ohne daß es, insbesondere bei divergierend eingesetzten Implantaten, zu Fehlbelastungen und damit zur Lockerung der Implantate kommt.

Obwohl das erfindungsgemäße Befestigungssystem gleichzeitig eine — wenn auch minimale — Beweglichkeit der Prothese zur Folge hat, entsteht für den Prothesenträger zu keiner Zeit das Gefühl der Unsicherheit.

Ein weiterer Vorteil des erfindungsgemäßen Befestigungssystems liegt darin, daß es einen Ausgleich der Resilienz der Schleimhaut ermöglicht, da die Prothese im Gegensatz zu den bisher bekannten Befestigungssystemen nicht permanent auf die Schleimhaut drückt.

Durch die vorliegende Erfindung wird es auch ermöglicht, auf Stegverbindungen zwischen den einzelnen Implantatpfosten zu verzichten, die vielfach dann angewendet wurden, wenn eine zusätzliche Fixierung der Gebißprothese erwünscht war. Der Nachteil dieser Stegkonstruktion bestand darin, daß sie bei einer einseitigen Belastung infolge ihres starren Verbundes eine gewisse Hebelwirkung auf benachbarte Implantatpfosten ausübten und deren Lockerung bewirken konnten.

Die Erfindung ermöglicht es auch, einen weiteren Nachteil auszugleichen, der bisher vielfach dann auftrat, wenn mehrere Implantate bzw. deren den Kieferkamm überragende Implantatpfosten als Befestigungsvorrichtung für eine herausnehmbare Gebißprothese dienten. Dieser Nachteil bestand darin, daß es in den seltensten Fällen gelang, die Implantate so genau auszurichten, daß die beim Kauen auftretenden Kräfte gleichmäßig auf die einzelnen Implantate übertragen wurden. Durch die Verwendung des erfindungsgemäßen Befestigungssystems besteht diese Gefahr nicht mehr und zwar auch dann nicht, wenn die beiden Implantate, die zur Aufnahme der kugelförmigen Haltevorrichtung vorgesehen sind, in voneinander divergierender Richtung eingesetzt sind. Die teilweise stattfindende Rotation der Prothese auf den Kugelköpfen hat dabei eine ausgleichende Wirkung. Es wird dadurch auch ermöglicht, die Kugelköpfe statt auf Implantaten auf natürlichen Zähnen, z. B. auf einzelstehenden unteren Eckzähnen anzubringen. Bisher bestanden hier große Probleme, wenn diese Zähne in divergierender Richtung standen und unter großen Schwierigkeiten eine genaue Anpassung des einzusetzenden Aufbaus vorgenommen werden mußte.

In einer besonders vorteilhaften Ausführungsform ist das erfindungsgemäße Befestigungssystem dadurch gekennzeichnet, daß die Kugelköpfe so ausgebildet sind, daß ihr Volumen zwischen 55 und 90 %, ganz besonders bevorzugt zwischen 65 und 85 % des vollen Kugelvolumens beträgt. Durch diese Art der Ausführung wird erreicht, daß der in die Mundhöhle ragende Teil niedriger ist als bei einer Vollkugel, um untersichgehende Teile zu vermeiden. Diese Ausbildung des Kugelkopfes ermöglicht auch einen besseren Abschluß gegenüber einer im Kiefer angelegten Öffnung. Zum anderen steht bei einer solchen Ausbildung des Kugelkopfes in jedem Fall eine ausreichend große Oberfläche zum Eingriff mit der Aufnahmekappe zur Verfügung.

In einer ganz besonders bevorzugten Ausführungsform sind die Kugelköpfe aus dichtgesintertem Aluminiumoxid einer Reinheit von mindestens 97 Gew.% hergestellt, da dieses Material bioinert ist und die Gefahr einer Zahnsteinbildung nicht gegeben ist. Aluminiumoxid ist auch deswegen ein bevorzugtes Material zur Herstellung der Kugelköpfe, weil es über eine hohe Verschleißfestigkeit verfügt und in Verbindung mit einer aus Kunststoff hergestellten Aufnahmekappe eine für den erfindungsgemäßen Zweck vorzügliche Reibungspaarung bildet.

Insbesondere, wenn die Oberfläche der Kugelköpfe so ausgebildet ist, daß sie einen Mittenrauhwert $R_a < 0{,}2$ μm aufweist, sind die Verschleißeigenschaften auf ein Minimum reduziert und wird zum anderen auch eine besonders gute Haftung zwischen den Kugelköpfen und den in die Gebißprothese eingesetzten Aufnahmekappen erreicht.

In einer bevorzugten Ausführungsform sind die Aufnahmekappen aus Polyvinylchlorid hergestellt, während die Adaptionsringe aus Polyäthylenvinylacetat bestehen. Durch diese Materialkombination wird in besonders vorteilhafter Weise erreicht, daß die Aufnahmekappen durch das elastischere Material der Adaptionsringe den Kugelköpfen im Bereich von deren Äquatoren anliegen und damit für eine gute Haftung zwischen Kugelkopf und den in der Gebißprothese befestigten Aufnahmekappen gesorgt ist. Hierdurch wird in besonders geeigneter Weise sichergestellt, daß die Prothese sich bei normaler horizontaler und vertikaler Belastung nicht von einem Kugelkopf abheben kann.

Zur Befestigung der Kugelköpfe haben sich mehrere Möglichkeiten als zweckmäßig erwiesen. Für die Befestigung der Kugelköpfe auf einem Implantat, wobei ganz besonders bevorzugt enossale Stiftimplantate sind, hat es sich als besonders vorteilhaft erwiesen, an den Kugelköpfen einen stiftförmigen Ansatz vorzunehmen. Gegebenenfalls kann an dessen unterem Ende ein Gewinde zur Verschraubung des Kugelkopfes auf dem Implantat vorgesehen sein. Diese Art der Befestigung ist deswegen besonders vorteilhaft, weil in verhältnismäßig einfacher Art und Weise ein Austausch des Kugelkopfes stattfinden kann.

Zur Fixierung eines Kugelkopfes mit Hilfe einer natürlichen Zahnwurzel hat es sich dagegen als zweckmäßig erwiesen, am Kugelkopf einen Stift vorzusehen, der mit Retentionshilfsmitteln versehen ist, die z. B. die Form von Wellen, Nuten, Kerben oder Unterschneidungen aufweisen. Diese Art von Retentionshilfsmitteln sorgt insbesondere dann, wenn in einer weiteren bevorzugten Ausführung auch der Stift aus nichtgesintertem Aluminiumoxid hergestellt ist, dafür, daß ein fester Sitz des Stiftes bzw. des Kugelkopfes mit dem sich neu bildenden Knochengewebe erzielt wird.

Das Einsetzen und Anpassen des erfindungsgemäßen Befestigungssystems wird nachfolgend an einem Beispiel beschrieben, bei dem die Kugelköpfe auf Schraubenimplantaten befestigt sind, ohne daß die Erfindung auf dieses Ausführungsbeispiel beschränkt ist. In gleicher Weise können statt der hier beschriebenen Schaubenimplantate auch andere enossale Implantate bzw. geeignete natürliche Zähne, insbesondere einzelstehende untere Eckzähne, zum Anbringen der erfindungsgemäßen Kugelköpfe verwendet werden. Das Beispiel betrifft eine Prothese für den Zahnersatz des Unterkiefers. Die Erfindung ist aber hierauf nicht beschränkt, sondern kann auch im Oberkiefer angewendet werden, wobei jedoch hervorzuheben ist, daß besondere Vorteile bei der Anwendung für den Unterkieferbereich bestehen.

Beispiel

Zunächst werden in an sich bekannter Weise zwei Schraubenimplantate zur Befestigung von zwei Kugelköpfen in den zahnlosen Unterkiefer eingesetzt. Anschließend wird mit Hilfe eines nachfolgend als Platzhalter bezeichneten Übertragungssystems ein Abdruck in an sich bekannter Weise hergestellt.

Die Platzhalter entsprechen in etwa den später einzusetzenden Kugelköpfen und werden in Öffnungen festgeschraubt, die in den über den Kiefer hinausragenden Kopfteilen der Implantate angebracht sind. Die Platzhalter unterscheiden sich in ihren äußeren Konturen jedoch insoweit von den Kugelköpfen, als sie in ihren Abmessungen um die Wandstärke von Aufnahmekappe und Adaptionsring vergrößert sind. Dieser Platzhalter hat die Funktion, in der Prothese den Raum freizuhalten, der später für das Einsetzen der Rotationskappe und des Adaptionsringes benötigt wird. Durch diese Ausbildung der Platzhalter wird dabei erreicht, daß im unteren Bereich, da wo die Adaptionsringe angebracht werden, in der Prothese Öffnungen freigehalten werden, die in ihren Abmessungen genau den Adaptionsringen entsprechen. Im oberen Teil dagegen, wo die Befestigung der Aufnahmekappen mit Hilfe eines Klebemittels in der Prothese erfolgt und wo das Maß der Platzhalter ebenfalls um die Wandstärke des Adaptionsringes vergrößert ist, bleibt durch diese Maßnahme der Raum frei, der zum Einbringen des Klebemittels benötigt wird. Als Material zu Herstellung der Platzhalter kann jedes beliebige, leicht bearbeitbare und formstabile Material verwendet werden. Als gut geeignet hat sich beispielsweise Messing erwiesen.

Nachdem mit Hilfe des in das Implantat eingesetzten Platzhalters ein Abdruck gemacht wurde, wird dieser vom Implantat entfernt und die Öffnung im Implantat bis zum Einsetzen des Kugelkopfes abgedichtet. Der aus den Implantaten entfernte Platzhalter wird in das Abdruckmodell eingesetzt und in bekannter Weise nach Anfertigung eines Positivmodells eine Prothese hergestellt.

Wenn der Einheilungsprozeß der Implantate nach ca. 6 Wochen abgeschlossen ist, werden die Dichtungen entfernt und die beiden Kugelköpfe aus dichtgesintertem Aluminiumoxid eingesetzt. Dies geschieht durch Verschraubung der stiftförmigen Ansätze, die auf ihrem einen Ende den Kugelkopf tragen und an ihrem anderen Ende ein Gewinde aufweisen. Nachdem die Kugelköpfe auf den Implantaten fixiert sind, wird auf die Kugelköpfe die inzwischen mit Hilfe des Abdrucks hergestellte Prothese aufgesetzt. In dieser befinden sich die bereits fest eingeklebten Aufnahmekappen und die die Aufnahmekappen umfassenden Adaptionsringe.

Weitere Einzelheiten und Vorteile der Erfindung ergeben sich aus der Beschreibung von beispielsweisen Ausführungsformen der Erfindung anhand der Figuren 1 bis 6.

Es zeigen:

Figur 1a im schematischen Schnitt nach Linie la-la der Fig. 1b eine Unterkieferprothese mit zwei Kugelköpfen in Aufnahmekappen,

Figur 1b ein Ausführungsbeispiel des erfindungsgemäßen Befestigungssystems, dargestellt an der Teilansicht einer Unterkieferprothese mit Aufnahmekappe, Adaptionsring und einem Kugelkopf, geschnitten in Ebene lb-lb der Fig. 1a,

Figur 2 eine vergrößerte Teilansicht zum Bereich II der Fig. 1b, darstellend Aufnahmekappe und Adaptionsring,

Figur 3 einen erfindungsgemäßen Kugelkopf, befestigt auf einem mit einem Gewinde versehenen Stiftimplantat,

Figur 4 einen in einem natürlichen Zahn befestigten Kugelkopf,

Figuren 5a und 5b das Zusammenwirken des Zahnersatzsystems bei divergierend eingesetzten Implantaten, am Beispiel einer Unterkieferprothese, einmal vor der Endmontage und einmal nach der Endmontage, geschnitten in Linie V-V der Fig. 1a,

Figur 6 die Funktion des erfindungsgemäßen Zahnersatzsystems, dargestellt in vereinfachter Form am Beispiel einer einseitig belasteten Unterkieferprothese.

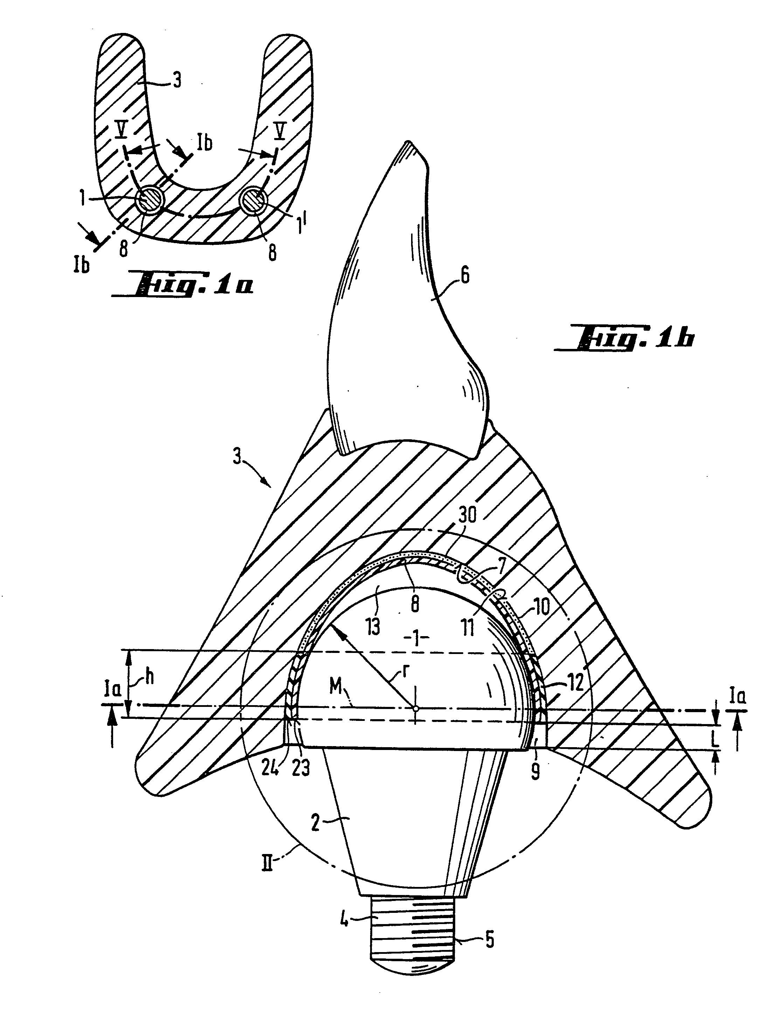

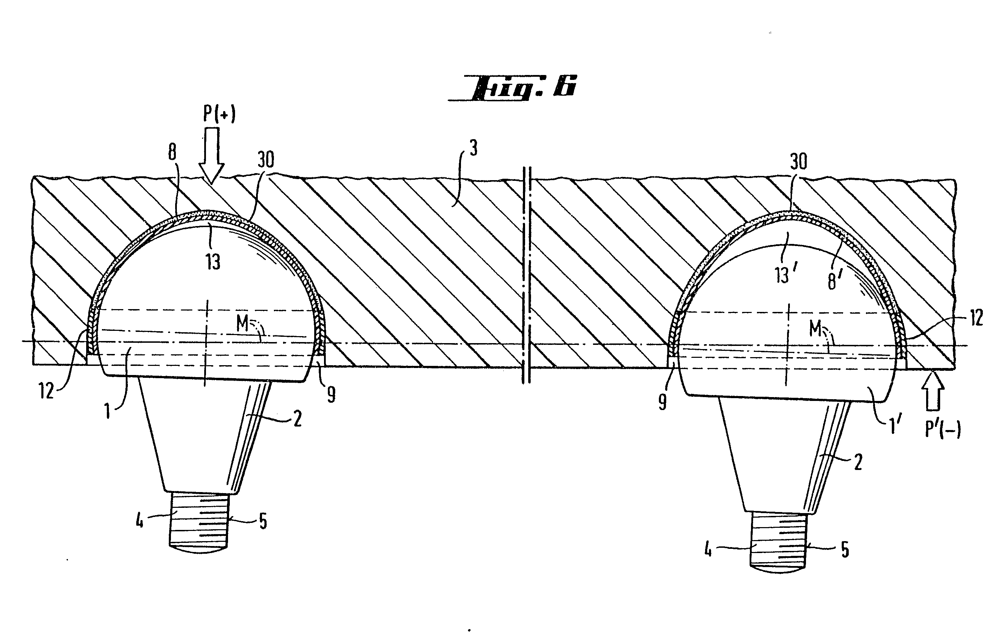

Figur 1b zeigt eine Teilansicht des erfindungsgemäßen Befestigungssystems, dargestellt am Beispiel einer Unterkieferprothese. Das gesamte System besteht aus einer Unterkieferprothese 3 für den totalen Zahnersatz und zwei zur Befestigung der Unterkieferprothese 3 vorgesehenen Kugelköpfen 1, von denen in Fig. 1b nur einer dargestellt ist. Der Kugelkopf 1 aus dichtgesintertem Aluminiumoxid mit einer Reinheit von 97,5 Gew.% und einem Mittenrauhwert $R_a = 0,03$, der auf einem stiftförmigen Ansatz 2 angebracht ist, an dessen unterem Ende 4 ein Gewinde 5 eingeschnitten ist, ist größer als halbkugelförmig ausgebildet und zwar beträgt sein Volumen 78,4 % des vollen Kugelvolumens. Auf der Unterkieferprothese 3 ist ein künstlicher Zahn 6 befestigt. In der Aushöhlung 7 der Unterkieferprothese 3 befindet sich eine aus Polyvinylchlorid hergestellte Aufnahmekappe 8, die dort mit einem kaltpolymerisierbaren Kunststoff 30 befestigt ist und dem Kugelkopf 1 im Bereich von dessen Äquator M anliegt. Im Öffnungsbereich 9 der Aushöhlung 7 befindet sich zwischen der Wand 10 der Aushöhlung 7 und der Wand 11 der Aufnahmekappe 8 ein Adaptionsring 12 aus Polyäthylenvinylacetat der — wie später noch in Fig. 6 gezeigt wird — bei einer Lageveränderung der Unterkieferprothese 3 für ein exaktes Anliegen der Aufnahmekappe 8 sorgt und infolgedessen eine stets gleichbleibende Friktion zwischen dem Kugelkopf 1 und der Aufnahmekappe 8 bewirkt.

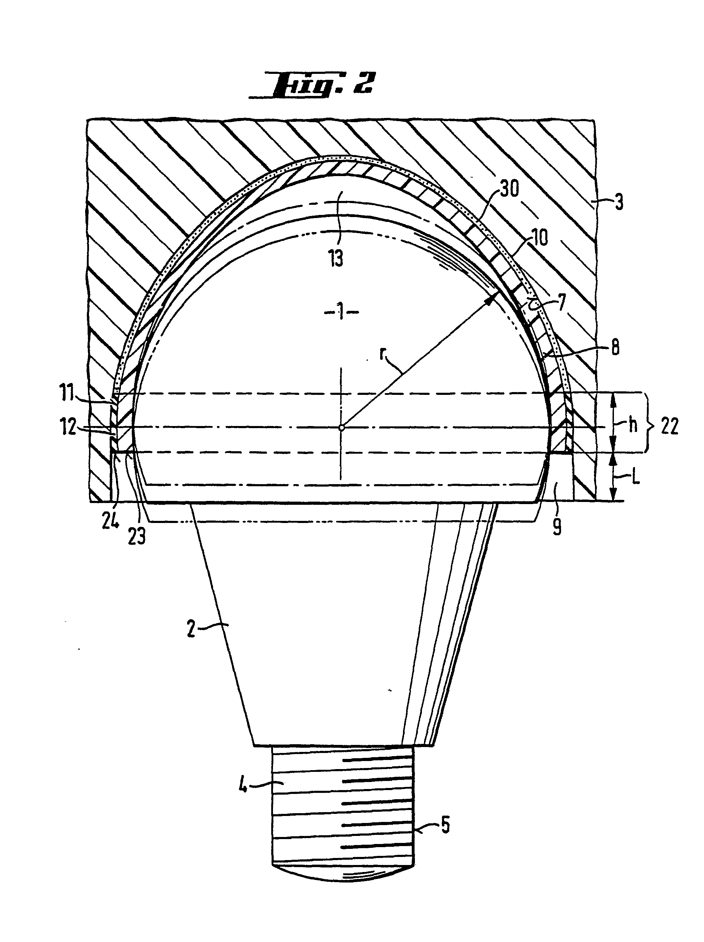

Wie aus Fig. 2 zu erkennen ist, ist die Aufnahmekappe 8 so ausgebildet, daß sie dem Kugelkopf 1 lediglich in dessen Äquatorbereich anliegt, während in ihrem oberen Bereich ein Hohlraum 13 frei bleibt. Bei ungleichmäßiger Belastung kann der Kugelkopf 1 in diesen Bereich eindringen. Bei der in Fig. 2 gezeigten Ausführungsform schließen Aufnahmekappe 8 und Adaptionsring 12 mit ihren unteren Kanten 23 und 24 bündig miteinander ab. Der Adaptionsring 12 ist in Fig. 1b so ausgebildet, daß das Verhältnis seiner Höhe h zum Halbmesser r des Kugelkopfes 1 1,5 : 2,5 beträgt.

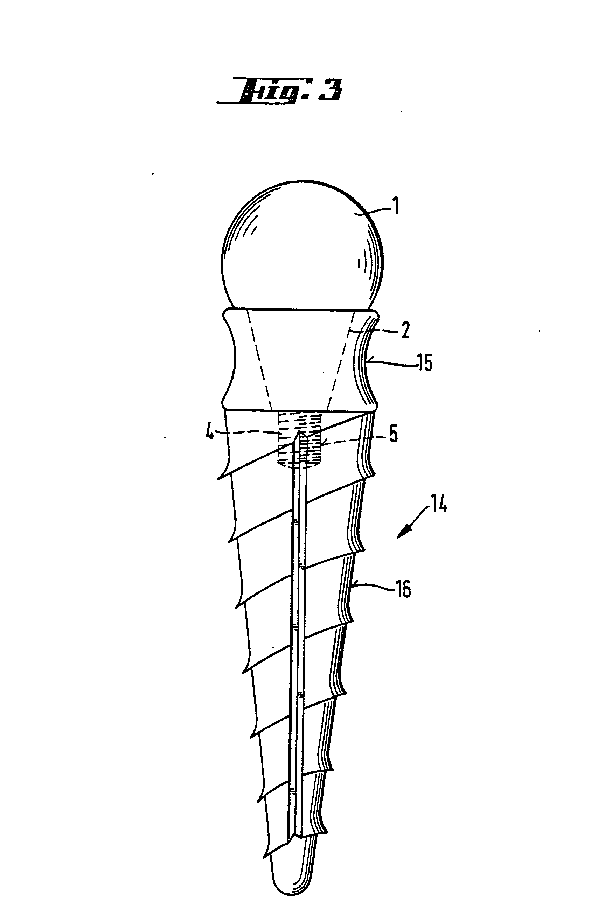

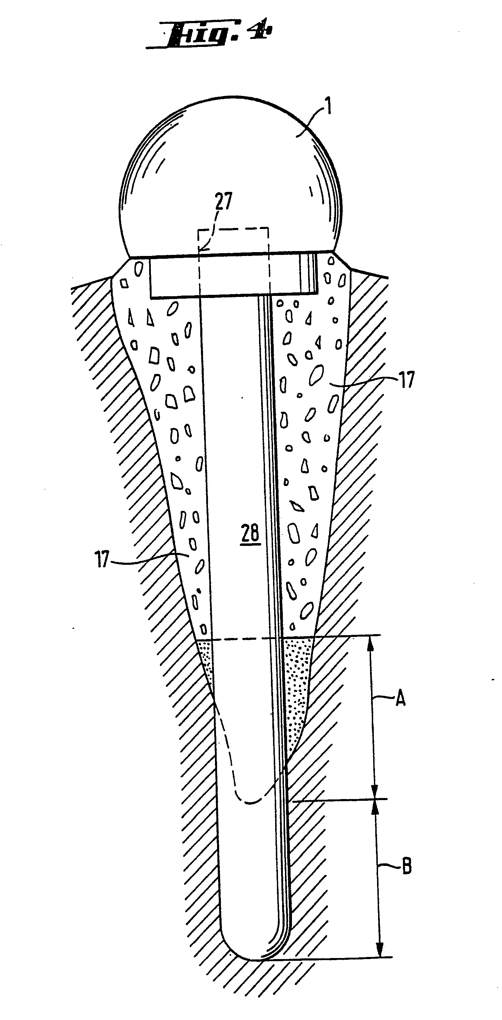

In Fig. 3 ist ein Kugelkopf 1 gezeigt, der mit seinem stiftförmigen Ansatz 2, an dessen unterem Ende 4 sich ein Gewinde 5 befindet, auf einem schraubenförmigen Implantat 14 aus dichtgesintertem Aluminiumoxid befestigt ist, das an seiner Außenseite ein Gewinde 16 aufweist. Einschraubwerkzeuge können kraftschlüssig am Kugelkopf 1 angesetzt werden. Zur Anlagerung der Epithelmanschette weist das Implantat 14 eine Hohlkehle 15 auf.

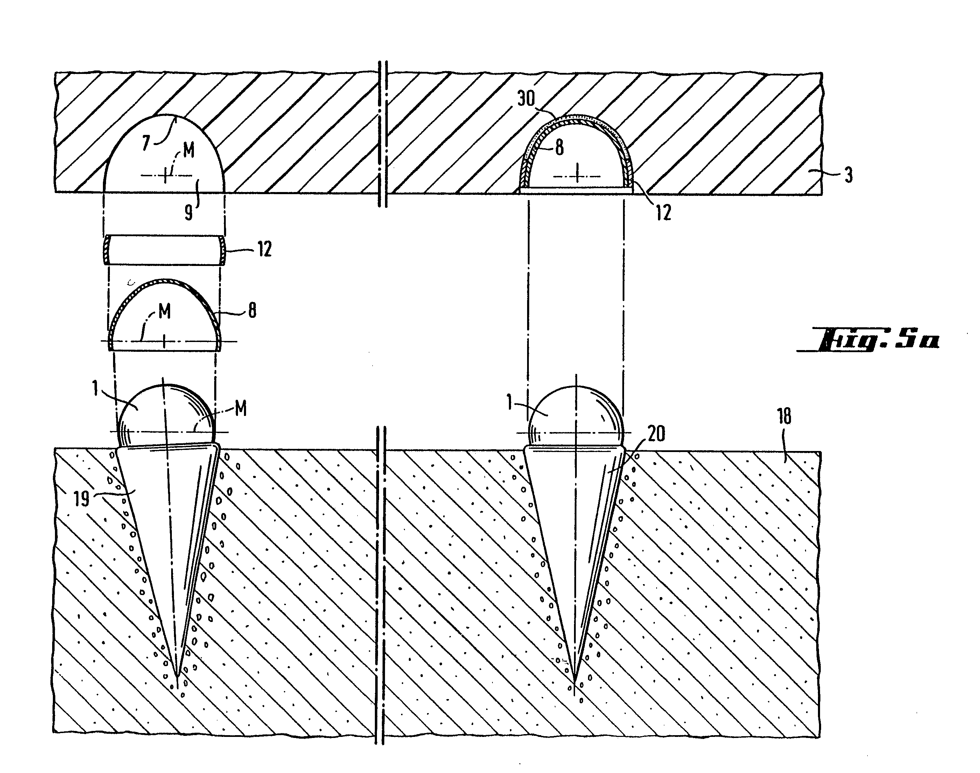

Fig. 4 zeigt einen Kugelkopf 1, der mit seinem Stift 28 in einer natürlichen Zahnwurzel 17 verankert ist. Der Stift 28 ist zylindrisch ausgebildet und in der Ausnehmung 27 des Kugelkopfes 1 durch nicht dargestellten Kitt befestigt. Beim Einsetzen des Kugelkopfes 1 mit seinem Stift 28 in die natürliche Zahnwurzel 17 wird in an sich bekannter Weise der Zahn zunächst gezogen und die Zahnwurzel um das Maß A verkürzt. Die Alveole wird um das Maß B vertieft und der Stift 28 der Zahnwurzel 17 bzw. der vertieften Alveole angepaßt. Dies geschieht durch Abschneiden des Stiftes 28 an seinem mit dem Kugelkopf 1 zu verbindenden Ende. Die Verklebung des Stiftes 28 erfolgt durch Verkleben mit Zement in der natürlichen Zahnwurzel 17.

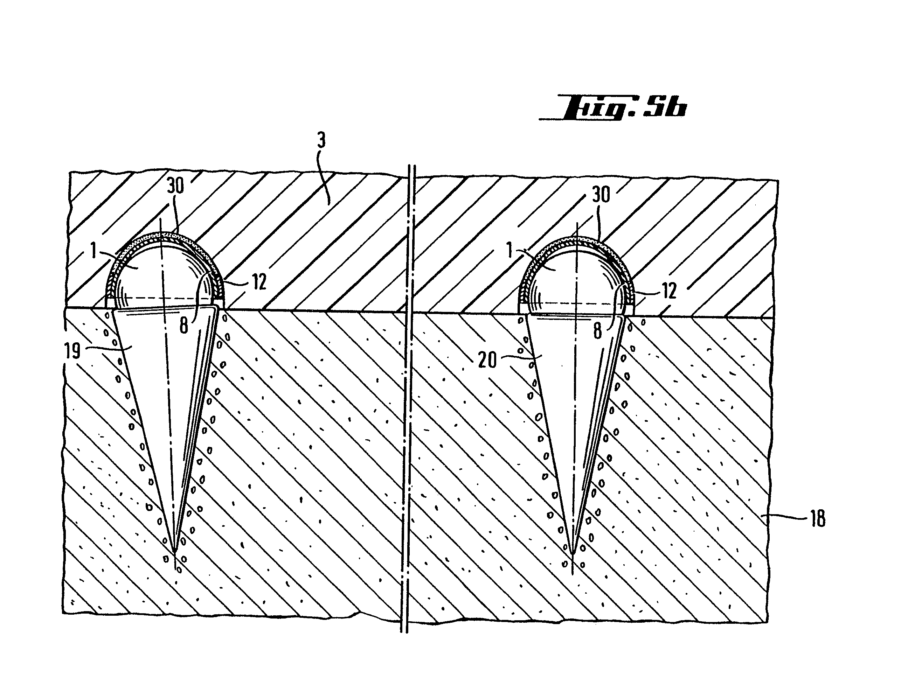

In Fig. 5a und 5b sind zwei in einem Unterkiefer 18 divergierend eingebrachte Implantate 19 und 20 dargestellt. Zum besseren Verständnis ist die Unterkieferprothese 3 in Fig. 5a von den Aufnahmekappen 8 abgehoben gezeigt. Die Figuren zeigen, daß auch trotz divergierend eingebrachter Implantate 19 und 20 die volle Funktionsfähigkeit des Zahnersatzsystems gewährleistet ist. Insbesondere zeigt Fig. 5b links, wie bei einem montierten Zahnersatzsystem Kugelkopf 1 und Aufnahmekappen 8 einen Ausgleich bei schief eingesetzten Implantaten bewirken.

Die Fig. 6 zeigt die Funktion des erfindungsgemäßen Befestigungssystems bei einseitiger Belastung. Da in der Aufnahmekappe 8 ein Hohlraum 13 ausgebildet ist, kann die Unterkieferprothese 3 bei starker einseitiger Belastung mit ihrer Aufnahmekappe 8 tiefer in den Kugelkopf 1 gepreßt werden. Gleichzeitig vergrößert sich der Hohlraum 13' durch die dort auftretende Abhebekraft P' (—), wie im rechten Teil von Fig. 6 gezeigt. Die Adaptionsringe 12 umgreifen beidseitig die Äquatore M und bewirken ein enges Anliegen der Aufnahmekappe 8', so daß ein Abheben vom Kugelkopf 1' angeschlossen ist und die Unterkieferprothese 3 ihren sicheren Sitz nicht verliert.

## Patentansprüche

1. Befestigungssystem für eine herausnehmbare schleimhautgetragene Prothese (3), bestehend aus zwei auf Implantaten (14, 19) oder natürlichen Zahnwurzeln (17) befestigten, das Volumen einer Halbkugel übertreffenden und mit einer hochglanzpolierten Oberfläche versehenen Kugelköpfen (1) zum Festhalten der Prothese (3), aus zwei zum Eingriff mit den Kugelköpfen (1) vorgesehenen, diesen angepaßten und in Aushöhlungen (7) der Prothese (3) befestigten Aufnahmekappen (8) aus glattem und elastischem Kunststoffmaterial und aus zwei im Bereich der Öffnung (9) der Aufnahmekappen (8) angeordneten Adaptionsringen (12) dadurch gekennzeichnet, daß die Aufnahmekappen (8) den Kugelköpfen (1) nur im Bereich von deren Äquatoren (M) anliegen und daß die Adaptionsringe (12) zwischen den Wänden (10) der Aushöhlungen (7) und den Aufnahmekappen (8) angeordnet sind und aus einem Kunststoffmaterial bestehen, das elastischer ist als das Material der Aufnahmekappen (8).

2. Befestigungssystem nach Anspruch 1, dadurch gekennzeichnet, daß jeder Kugelkopf (1) so ausgebildet ist, daß sein Volumen zwischen 55 % und 90 % des vollen Kugelvolumens beträgt.

3. Befestigungssystem nach einem der Ansprüche 1 und 2, dadurch gekennzeichnet, daß der Adaptionsring (12) so ausgebildet ist, daß das Verhältnis seiner Höhe (h) zum Halbmesser (r) des Kugelkopfes (1) zwischen 1 : 2,5 und 2 : 2,5 beträgt.

4. Befestigungssystem nach einem der Ansprüche 1 bis 3, dadurch gekennzeichnet, daß die Aufnahmekappe (8) mittels eines kaltpolymerisierenden Klebemittels (30) an der Wand (10) der Aushöhlung (7) so befestigt ist, daß zumindest der zum Einsetzen des Adaptionsringes (12) vorgesehene Bereich (22) von Klebemittel frei bleibt.

5. Befestigungssystem nach einem der Ansprüche 1 bis 4, dadurch gekennzeichnet, daß die untere Kante (23) der Aufnahmekappe (8) mit der Unterkante (24) des Adaptionsringes (12) bündig abschließt.

6. Befestigungssystem nach einem der Ansprüche 1 bis 5, dadurch gekennzeichnet, daß die Wand (10) der Aushöhlung (7) die bündig abschliessenden Unterkanten (23) und (24) der Aufnahmekappe (8) und des Adaptionsringes (12) um das Maß (L) überragt, das 1/5 bis 3/5 des Halbmessers (r) des Kugelkopfes (1) beträgt.

7. Befestigungssystem nach einem der Ansprüche 1 bis 6, dadurch gekennzeichnet, daß der Kugelkopf (1) aus dicht gesintertem Aluminiumoxid einer Reinheit von mindestens 97 Gew.% besteht.

8. Befestigungssystem nach einem der Ansprüche 1 bis 7, dadurch gekennzeichnet, daß die Oberfläche des Kugelkopfes (1) einen Mittenrauhwert $R_a < 0,2$ μm aufweist.

9. Befestigungssystem nach einem der Ansprüche 1 bis 8, dadurch gekennzeichnet, daß die Aufnahmekappe (8) aus Polyvinylchlorid besteht.

10. Befestigungssystem nach einem der Ansprüche 1 bis 9, dadurch gekennzeichnet, daß der Adaptionsring (12) aus Polyäthylenvinylacetat besteht.

11. Befestigungssystem nach einem der Ansprüche 1 bis 10, dadurch gekennzeichnet, daß der Kugelkopf (1) einen Stift (28) oder einen stiftförmigen Ansatz (2) aus gesintertem Aluminiumoxid aufweist.

12. Befestigungssystem nach einem der Ansprüche 1 bis 11, dadurch gekennzeichnet, daß der Kugelkopf (1) einen stiftförmigen Ansatz (2) aufweist, der an seinem dem Kugelkopf (1) gegenüberliegenden Ende (4) konisch zuläuft.

13. Befestigungssystem nach einem der Ansprüche 1 bis 11, dadurch gekennzeichnet, daß der Kugelkopf (1) einen für die Fixierung des Kugelkopfes (1) vorgesehenen zylindrischen Stift aufweist.

14. Befestigungssystem nach einem der Ansprüche 1 bis 13, dadurch gekennzeichnet, daß der Kugelkopf (1) auf einem eine zylindrische Form aufweisenden Stiftimplantat angeordnet ist.

## Claims

1. Fastening system for a removable prosthesis (3) which is supported by the mucous membrane, comprising two spherical heads (1) for securing the prosthesis (3), which are fastened to implants (14, 19) or to natural tooth roots (17), are greater in volume than a hemisphere and are provided with a highly polished surface, two receiving caps (8) comprising smooth and resilient plastics material that are provided to engage the spherical heads (1), are matched to the latter and are secured in recesses (7) in the prosthesis (3), and two adaption rings (12) arranged in the region of the opening (9) in the receiving caps (8), characterised in that the receiving caps (8) rest against the spherical heads (1) only in the region of their equators (M) and that the adaption rings (12) are located between the walls (10) of the recesses (7) and the receiving caps (8) and comprise a plastics material that is more resilient than the material of the receiving caps (8).

2. Fastening system according to claim 1, characterised in that each spherical head (1) is so constructed that its volume is between 55 % and 90 % of the volume of a complete sphere.

3. Fastening system according to claim 1 or 2, characterised in that the adaption ring (12) is so constructed that the ratio of its height (h) to the radius (r) of the spherical head (1) is between 1 : 2.5 and 2 : 2.5.

4. Fastening system according to any one of claims 1 to 3, characterised in that the receiving cap (8) is fastened to the wall (10) of the recess (7) by means of a cold-polymerising adhesive (30) in such a manner that at least that area (22) that is provided for inserting the adaption ring (12) remains free of adhesive.

5. Fastening system according to any one of claims 1 to 4, characterised in that the lower edge (23) of the receiving cap (8) terminates flush with the lower edge (24) of the adaption ring (12).

6. Fastening system according to any one of claims 1 to 5, characterised in that the wall (10) of the recess (7) projects beyond the flush lower edges (23) and (24) of the receiving cap (8) and of the adaption ring (12) by a distance (L) which is from 1/5 to 3/5 of the radius (r) of the spherical head (1).

7. Fastening system according to any one of claims 1 to 6, characterised in that the spherical head (1) comprises densely sintered aluminium oxide of a purity of at least 97 % by weight.

8. Fastening system according to any one of claims 1 to 7, characterised in that the surface of the spherical head (1) has a mean roughness value of $R_a < 0.2 \mu m$.

9. Fastening system according to any one of claims 1 to 8, characterised in that the receiving cap (8) comprises polyvinyl chloride.

10. Fastening system according to any one of claims 1 to 9, characterised in that the adaption ring (12) comprises polyethylene vinyl acetate.

11. Fastening system according to any one of claims 1 to 10, characterised in that the spherical head (1) has a peg (28) or a peg-like projection (2) made of sintered aluminium oxide.

12. Fastening system according to any one of claims 1 to 11, characterised in that the spherical head (1) has a peg-like projection (2) which tapers conically at its end (4) opposite the spherical head (1).

13. Fastening system according to any one of claims 1 to 11, characterised in that the spherical head (1) has a cylindrical peg for the purpose of fixing the spherical head (1).

14. Fastening system according to any one of claims 1 to 13, characterised in that the spherical head (1) is located on a peg implant that is cylindrical in shape.

## Revendications

1. Système de fixation pour une prothèse amovible (3) supportée par la muqueuse, constitué par deux têtes sphériques (1) destinées à maintenir la prothèse (3), fixées sur des implants (14, 19) ou sur des racines naturelles (17) des dents, dépassant le volume d'une hémisphère et munies d'une surface polie miroir par deux calottes réceptrices (8) en matériau synthétique lisse et élastique, prévues pour venir en contact avec les têtes sphériques (1), adaptées à celles-ci et fixées dans des évidements (7) de la prothèse (3) et par deux bagues d'adaptation (12) placées dans la zone de l'ouverture (9) des calottes réceptrices (8), caractérisé par le fait que les calottes réceptrices (8) ne sont appliquées sur les têtes sphériques (1) que dans la zone de leurs équateurs (M) et que les bagues d'adaptation (12) sont disposées entre les parois (10) des évidements (7) et les calottes réceptrices (8) et sont réalisées en un matériau synthétique qui est plus élastique que le matériau des calottes réceptrices (8).

2. Système de fixation selon la revendication 1, caractérisé par le fait que chaque tête sphérique (1) est conçue de façon que son volume représente entre 55 % et 90 % du volume de la sphère entière.

3. Système de fixation selon l'une des revendications 1 et 2, caractérisé par le fait que la bague d'adaptation (12) est conçue de façon que le rapport entre sa hauteur (h) et le diamètre (r) de la tête sphérique (1) se situe entre 1 : 2,5 et 2 : 2,5.

4. Système de fixation selon l'une des revendications 1 à 3, caractérisé par le fait que la calotte réceptrice (8) est fixée sur la paroi (10) de l'évidement (7) au moyen d'un adhésif (3a) polymérisant à froid de façon qu'au moins la zone (22) prévue pour l'insertion de la bague d'adaptation (12) reste exempte d'adhésif.

5. Système de fixation selon l'une des revendications 1 à 4, caractérisé par le fait que l'arête inférieure (23) de la calotte réceptrice (8) est dans l'alignement de l'arête inférieure (24) de la bague d'adaptation (12).

6. Système de fixation selon l'une des revendications 1 à 5, caractérisé par le fait que la paroi (10) de l'évidement (7) dépasse des arêtes inférieures (23) et (24), situées dans l'alignement l'une de l'autre, de la calotte réceptrice (8) et de la bague d'adaptation (12) sur une distance L qui représente 1/5 à 3/5 du diamètre (r) de la tête sphérique (1).

7. Système de fixation selon l'une des revendications 1 à 6, caractérisé par le fait que la tête sphérique (1) est réalisée en oxyde d'aluminium fritté à densité maximale d'une pureté d'au moins 97 % en poids.

8. Système de fixation selon l'une des revendications 1 à 7, caractérisé par le fait que la surface de la tête sphérique (1) présente une rugosité moyenne arithmétique $R_a < 0,2 \mu m$.

9. Système de fixation selon l'une des revendications 1 à 8, caractérisé par le fait que la calotte réceptrice (8) est réalisée en chlorure de polyvinyle.

10. Système de fixation selon l'une des revendications 1 à 9, caractérisé par le fait que la bague d'adaptation (12) est réalisée en poly(éthylène/acétate de vinyle).

11. Système de fixation selon l'une des revendications 1 à 10, caractérisé par le fait que la tête sphérique (1) comporte une cheville (28) ou un appendice (2) en forme de cheville en oxyde d'aluminium fritté.

12. Système de fixation selon l'une des revendications 1 à 11, caractérisé par le fait que la tête sphérique (1) comporte un appendice (2) en forme de cheville qui se termine en cône au niveau de son extrémité (4) opposée à la tête sphérique (1).

13. Système de fixation selon l'une des revendications 1 à 11, caractérisé par le fait que la tête sphérique (1) comporte une cheville cylindrique prévue pour la fixation de la tête sphérique (1).

14. Système de fixation selon l'une des revendications 1 à 13, caractérisé par le fait que la tête sphérique (1) est placée sur un implant en forme de cheville présentant une forme cylindrique.

**Fig. 1a**

**Fig. 1b**

Fig. 2

# Fig. 3

*Fig. 4*

Fig. 5a

**Fig.5b**

*Fig. 6*